# EUROPEAN PATENT APPLICATION

(11) **EP 3 251 687 A1**
(43) Date of publication of application: **06.12.2017**
(21) Application number: 16172416.6
(22) Date of filing: 01.06.2016
(51) Int. Cl.: A61K 38/08, A61K 38/10, C07K 7/06, C07K 7/08, A61P 3/08, A61P 3/10

(54) **BIOACTIVE PEPTIDES FOR BLOOD GLUCOSE CONTROL**

(71) Applicant: Centrum Innowacji Edoradca Sp. z o.o. Spolka Komandytowa, 83-110 Tczew (PL)
(72) Inventor: PIERZYNOWSKI, Stefan, 23132 Trelleborg (SE); GONCHAROVA, Kateryna, 23132 Trelleborg (SE)
(74) Representative: Novitas Patent AB

(57) **Abstract**

Peptides or peptidomimetics with amylase-derived sequence effective in regulating glucose metabolism, and related compositions. Therapeutic uses thereof in hyperglycemic conditions, conditions involving hyperinsulinemia and conditions involving impaired insulin sensitivity, such as type 1 diabetes, type 2 diabetes, type 3 diabetes, metabolic syndrome, prediabetes, obesity, Alzheimer's disease, Cushing's syndrome.

## Description

### TECHNICAL FIELD

The present invention relates to the field of means and methods for controlling blood glucose in a subject, in particular with regard to treatment of hyperglycemic conditions, conditions involving hyperinsulinemia and conditions involving impaired insulin sensitivity.

### BACKGROUND TO THE INVENTION

Insulin misregulation in adolescence has a considerable impact on metabolic processes in adults. High carbohydrate consumption and as well as high protein diet results especially when combined with physical inactivity in the overproduction of insulin, leading to the development of insulin resistance, undesirable anabolic activity (including weight gain and obesity), metabolic syndrome and ultimately type 2 diabetes. Existing mainstream treatments for type 2 diabetes include exogenous insulin administration, GLP-1 receptor agonists, metformin, sulfonylureas, meglitinides, DPP-4 inhibitors, thiazolidinediones and SGLT2 inhibitors. Other treatments include exercise and dietary management. None of the existing treatments is without drawbacks and the treatments are not fully efficacious in a substantial number of patients.

The role of the exocrine pancreas in food digestion and nutrient breakdown in the intestine is well-studied and recognized. For a long time, the activity of gut pancreatic enzymes has generally been considered merely a source of the postprandial hyperglycemic condition, which is reflected in studies of different inhibitors on its actions (Bays 2004; McDougall and Stewart 2005). However, alternative aspects of pancreatic enzyme impact on glycemic control and insulin regulation seem to have been mostly overlooked. The control of glucose in the blood is believed to be accomplished by the concerted action of endocrine pancreas, liver, kidneys, skeletal muscle and gastrointestinal hormones acting on insulin secretion.

Glucose intolerance associated with a delayed insulin release has been observed during *exocrine* pancreatic insufficiency (i.e. in a condition characterized by lack on pancreatic enzyme secretion) in humans and pigs (Knop, et al. 2007; Lozinska, et al. 2013). So far, such physiological changes are recognized as a pancreatogenic type of diabetes (3c type) and suggested to occur together with exocrine pancreatic insufficiency or pancreas injury (Ewald and Hardt 2013).

In humans, the influence of pancreatic enzymes replacement therapy on glucose metabolism remains controversial although impaired fasting glucose was found in patients with exocrine and/or endocrine pancreatic insufficiency. On one hand, improved blood glucose control and reduced glycated hemoglobin were reported in one study (Knop, et al. 2007; Mohan, et al. 1998), where on the other hand other studies found no effect (Ewald, et al. 2007; Glasbrenner, et al. 1990; O'Keefe, et al. 2001).

By using young pigs with induced exocrine pancreatic insufficiency, the inventors found in a previous study that pancreatic enzymes present in the gut lumen improved direct blood glucose utilization (without reinforcing insulin release) and improved growth performance of the pigs (Lozinska, et al. 2013). Furthermore, utility of amylase in the treatment of diabetes has been mentioned in patent literature in WO2006136161 and GB2468629.

An object of the present invention is to provide improved methods and means for blood glucose control, in particular with regard to insulin sensitivity. Another object of the present invention is to provide improved methods and means for the treatment of hyperglycemic conditions, conditions involving hyperinsulinemia and for conditions involving impaired insulin sensitivity.

### DEFINITIONS

The term *amylase* refers to an enzyme having alpha-amylase activity (EC 3.2.1.1).

The term *treatment* in the present context refers to treatments resulting in a beneficial effect on a subject afflicted with the condition to be treated, including any degree of alleviation, including minor alleviation, substantial alleviation, major alleviation as well as cure. Preferably, the degree of alleviation is at least a minor alleviation.

The term *prevention* in the present context refers to preventive measures resulting in any degree of reduction in the likelihood of developing the condition to be prevented, including a minor, substantial or major reduction in likelihood of developing the condition as well as total prevention. Preferably, the degree of likelihood reduction is at least a minor reduction.

### BRIEF DESCRIPTION OF THE FIGURES

**Figure 1** illustrates the effect of a specific set of amylase-derived peptides compared to intact porcine amylase and no treatment in intraduodenal glucose tolerance test (ID.GTT) on glucose measured in **jugular** blood. Figure 1a: DJB - bariatric pigs. Figure 1b: Control - normal pigs.
**Figure 2** illustrates the effect of a specific set of amylase-derived peptides compared to intact porcine amylase and no treatment in intraduodenal glucose tolerance test (ID.GTT) on glucose measured in **portal** blood. Figure 2a: DJB - bariatric pigs. Figure 2b: Control - normal pigs.

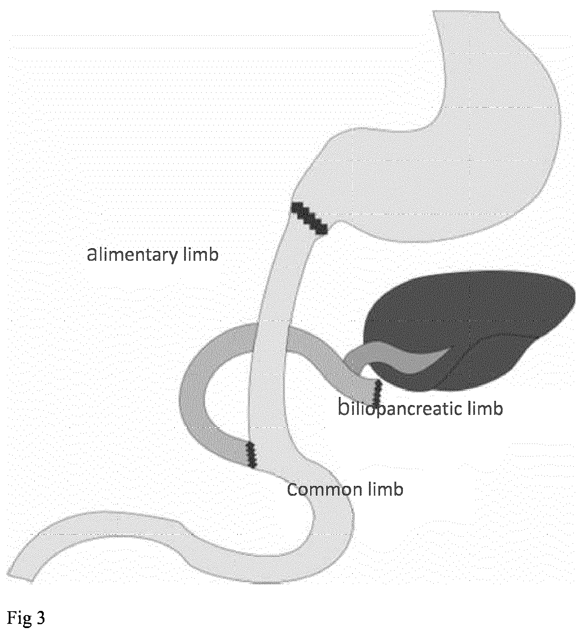

**Figure 3****:** Illustration of the bariatric operation model.

### SUMMARY OF THE INVENTION

The present invention relates to the following items. The subject matter disclosed in the items below should be regarded disclosed in the same manner as if the subject matter were disclosed in patent claims.
1. An isolated peptide or peptidomimetic of no more than 20 residues,
   comprising an amylase sequence selected from:
   a. GNENEFR (SEQ ID NO: 1);
   b. SEQ ID NO: 1 with 4N to D substitution;
   c. TSIVHLFEWR (SEQ ID NO: 2);
   d. SISNSAEDPF (SEQ ID NO: 3);
   e. VNGEDVNDW (SEQ ID NO: 4);
   f. SEQ ID NO: 4 with 1V to F and/or 4E to N substitution;
   g. IGPPNNNGVIK (SEQ ID NO: 5);
   h. a sequence having 1, 2, 3 or 4 differences from any of SEQ ID NOs:1-5, wherein the differences are residue substitutions; and
   i. a sequence having 1, 2, 3, 4, 5 or 6 differences from any of SEQ ID NOs:1-5, wherein the differences are independently selected from residue substitutions, residue deletions and residue insertions internal to the sequence of any of SEQ ID NOs: 1-5.
2. The peptide or peptidomimetic according to item 1, wherein the amylase sequence is a sequence having 1, 2 or 3 differences from any of SEQ ID NOs:1-5, wherein the differences are independently selected from residue substitutions, residue deletions and residue insertions internal to the sequence of any of SEQ ID NOs: 1-5.
3. The peptide or peptidomimetic according to any of items 1-2, wherein the amylase sequence is a sequence having 1, 2 or 3 differences from any of SEQ ID NOs:1-5, wherein the differences are independently selected from residue deletions and residue insertions internal to the sequence of any of SEQ ID NOs: 1-5.
4. The peptide or peptidomimetic according to any of items 1-3, wherein said amylase sequence has a sequence differing from any of SEQ ID NO: 1-5 by alanine substitutions numbering 1, 2, 3, 4, 5 or 6 in total.
5. The peptide or peptidomimetic according to any of items 1-4, wherein the amylase sequence is selected from:
   a. GNENEFR (SEQ ID NO: 1);
   b. SEQ ID NO: 1 with 4N to D substitution;
   c. a sequence having 1, 2, 3 or 4 differences from SEQ ID NO:1, wherein the differences are residue substitutions; and
   d. a sequence having 1, 2, 3, 4, 5 or 6 differences from SEQ ID NO:1, wherein the differences are independently selected from residue substitutions, residue deletions and residue insertions internal to the sequence of SEQ ID NO:1.
6. The peptide or peptidomimetic according to any of items 1-4, wherein the amylase sequence is selected from:
   a. TSIVHLFEWR (SEQ ID NO: 2);
   b. a sequence having 1, 2, 3 or 4 differences from SEQ ID NO: 2, wherein the differences are residue substitutions; and
   c. a sequence having 1, 2, 3, 4, 5 or 6 differences from SEQ ID NO: 2, wherein the differences are independently selected from residue substitutions, residue deletions and residue insertions internal to the sequence of SEQ ID NO:2.
7. The peptide or peptidomimetic according to any of items 1-4, wherein the amylase sequence is selected from:
   a. SISNSAEDPF (SEQ ID NO: 3);
   b. a sequence having 1, 2, 3 or 4 differences from SEQ ID NO: 3, wherein the differences are residue substitutions; and
   c. a sequence having 1, 2, 3, 4, 5 or 6 differences from SEQ ID NO: 3, wherein the differences are independently selected from residue substitutions, residue deletions and residue insertions internal to the sequence of SEQ ID NO: 3.
8. The peptide or peptidomimetic according to any of items 1-4, wherein the amylase sequence is selected from:
   a. VNGEDVNDW (SEQ ID NO: 4);
   b. SEQ ID NO: 4 with 1V to F and/or 4E to N substitution;
   c. a sequence having 1, 2, 3 or 4 differences from SEQ ID NO: 4, wherein the differences are residue substitutions; and
   d. a sequence having 1, 2, 3, 4, 5 or 6 differences from SEQ ID NO: 4, wherein the differences are independently selected from residue substitutions, residue deletions and residue insertions internal to the sequence of SEQ ID NO: 4.
9. The peptide or peptidomimetic according to any of items 1-4, wherein the amylase sequence is selected from:
   a. IGPPNNNGVIK (SEQ ID NO: 5);
   b. a sequence having 1, 2, 3 or 4 differences from SEQ ID NO:5, wherein the differences are residue substitutions; and
   c. a sequence having 1, 2, 3, 4, 5 or 6 differences from SEQ ID NO:5, wherein the differences are independently selected from residue substitutions, residue deletions and residue insertions internal to the sequence of SEQ ID NO:5.
10. The peptide or peptidomimetic according to item 1, wherein the amylase sequence is GNENEFR (SEQ ID NO:1) or SEQ ID NO: 1 with substitution of 4N to D.
11. The peptide or peptidomimetic according to item 1, wherein the amylase sequence is TSIVHLFEWR (SEQ ID NO: 2).
12. The peptide or peptidomimetic according to item 1, wherein the amylase sequence is SISNSAEDPF (SEQ ID NO: 3).
13. The peptide or peptidomimetic according to item 1, wherein the amylase sequence is VNGEDVNDW (SEQ ID NO: 4), or SEQ ID NO: 4 with 1V to F and/or 4E to N substitution.
14. The peptide or peptidomimetic according to item 1, wherein the amylase sequence is IGPPNNNGVIK (SEQ ID NO: 5).
15. The peptide or peptidomimetic according to item 1, wherein the sequence of the peptide or peptidomimetic consists of GNENEFR (SEQ ID NO:1) or SEQ ID NO: 1 with 4N to D substitution.
16. The peptide or peptidomimetic according to item 1, wherein the sequence of the peptide or peptidomimetic consists of TSIVHLFEWR (SEQ ID NO: 2).
17. The peptide or peptidomimetic according to item 1, wherein the sequence of the peptide or peptidomimetic consists of SISNSAEDPF (SEQ ID NO: 3).
18. The peptide or peptidomimetic according to item 1, wherein the sequence of the peptide or peptidomimetic consists of VNGEDVNDW (SEQ ID NO: 4), or SEQ ID NO: 4 with 1V to F and/or 4E to N substitution.
19. The peptide or peptidomimetic according to item 1, wherein the sequence of the peptide or peptidomimetic consists of IGPPNNNGVIK (SEQ ID NO: 5).
20. The peptide or peptidomimetic according to any of items 1-19, comprising no more than 15 residues, preferably no more than 12.
21. The peptide or peptidomimetic according to any of items 1-20, being a peptide.
22. The peptide or peptidomimetic according to any of items 1-20, being a peptidomimetic.
23. The peptide or peptidomimetic according to any of items 1-22, having a modified C-terminal, preferably an amidated C-terminal.
24. The peptide or peptidomimetic according to any of items 1-23, having a modified N-terminal, preferably an acylated N-terminal.
25. The peptide or peptidomimetic according to any of items 1-24, having a cyclic backbone.
26. The peptide or peptidomimetic according to any of items 1-25, comprising one or more non-natural residues.
27. The peptide or peptidomimetic according to any of items 1-26, comprising one or more D-amino acid residues.
28. The peptide or peptidomimetic according to any of items 1-27, comprising one or more non-natural bonds in place of peptide bonds.
29. The peptide or peptidomimetic according to any of items 1-28, being a peptide and comprising at least one difference compared to any naturally occurring peptide.
30. The peptide or peptidomimetic according to any of items 1-29, being a peptide wherein the peptide sequence comprises at least one difference compared to any of the sequences according to SEQ ID NO: 1-5.
31. The peptide or peptidomimetic according to any of items 1-30, wherein the peptide or peptidomimetic is effective in regulating glucose homeostasis in a mammal.
32. The peptide or peptidomimetic according to any of items 1-31, for use as a medicament.
33. The peptide or peptidomimetic according to any of items 1-32, for use in the treatment or prevention of condition selected from the list consisting of hyperglycemic conditions, conditions involving hyperinsulinemia and conditions involving impaired insulin sensitivity.
34. The peptide or peptidomimetic according to item 33, wherein the condition is selected from the list consisting of type 1 diabetes, type 2 diabetes, type 3 diabetes, metabolic syndrome, prediabetes, obesity, Alzheimer's disease and Cushing's syndrome.
35. A composition for oral use, comprising 1, 2, 3, 4, 5 or more peptides or peptidomimetics according to any of items 1-34.
36. The composition according to item 35, comprising a peptide or peptidomimetic according to item 5 or any item dependent thereon.
37. The composition according to any of items 35-36, comprising a peptide or peptidomimetic according to item 6 or any item dependent thereon.
38. The composition according to any of items 35-37, comprising a peptide or peptidomimetic according to item 7 or any item dependent thereon.
39. The composition according to any of items 35-38, comprising a peptide or peptidomimetic according to item 8 or any item dependent thereon.
40. The composition according to any of items 35-39, comprising a peptide or peptidomimetic according to item 9 or any item dependent thereon.
41. The composition according to any of items 35-40, comprising the following five peptides or peptidomimetics:
   a. a peptide or peptidomimetic according to item 5 or any item dependent thereon;
   b. a peptide or peptidomimetic according to item 6 or any item dependent thereon;
   c. a peptide or peptidomimetic according to item 7 or any item dependent thereon;
   d. a peptide or peptidomimetic according to item 8 or any item dependent thereon; and
   e. a peptide or peptidomimetic according to item 9 or any item dependent thereon.
42. The composition according to any of items 35-41, comprising a peptide or peptidomimetic comprising an amylase sequence GNENEFR (SEQ ID NO:1) or SEQ ID NO: 1 with substitution of 4N to D.
43. The composition according to any of items 35-42, comprising a peptide or peptidomimetic comprising an amylase sequence TSIVHLFEWR (SEQ ID NO: 2).
44. The composition according to any of items 35-43, comprising a peptide or peptidomimetic comprising an amylase sequence SISNSAEDPF (SEQ ID NO: 3).
45. The composition according to any of items 35-44, comprising a peptide or peptidomimetic comprising an amylase sequence VNGEDVNDW (SEQ ID NO: 4), or SEQ ID NO: 4 with 1V to F and/or 4E to N substitution.
46. The composition according to any of items 35-45, comprising a peptide or peptidomimetic comprising an amylase sequence IGPPNNNGVIK (SEQ ID NO: 5).
47. The composition according to any of items 35-46, comprising a peptide or peptidomimetic the sequence of which consists of GNENEFR (SEQ ID NO:1) or SEQ ID NO: 1 with 4N to D substitution.
48. The composition according to any of items 35-47, comprising a peptide or peptidomimetic, the sequence of which consists of TSIVHLFEWR (SEQ ID NO: 2).
49. The composition according to any of items 35-48, comprising a peptide or peptidomimetic, the sequence of which consists of SISNSAEDPF (SEQ ID NO: 3).
50. The composition according to any of items 35-49, comprising a peptide or peptidomimetic, the sequence of which consists of VNGEDVNDW (SEQ ID NO: 4), or SEQ ID NO: 4 with 1V to F and/or 4E to N substitution.
51. The composition according to any of items 35-50, comprising a peptide or peptidomimetic, the sequence of which consists of IGPPNNNGVIK (SEQ ID NO: 5).
52. The composition according to item 35, wherein the composition comprises the following peptides:
   a. a peptide, the sequence of which consists of GNENEFR (SEQ ID NO:1) or SEQ ID NO: 1 with substitution of 4N to D;
   b. a peptide, the sequence of which consists of TSIVHLFEWR (SEQ ID NO: 2);
   c. a peptide, the sequence of which consists of SISNSAEDPF (SEQ ID NO: 3);
   d. a peptide, the sequence of which consists of VNGEDVNDW (SEQ ID NO: 4) or SEQ ID NO: 4 with 1V to F and/or 4E to N substitution; and
   e. a peptide, the sequence of which consists of IGPPNNNGVIK (SEQ ID NO: 5).
53. The composition according to any of items 35-52, wherein the composition is formulated for duodenal release.
54. The composition according to any of items 35-53, for use as a medicament.
55. The peptide or peptidomimetic according to any of items 35-54, for use in the treatment or prevention of condition selected from the list consisting of hyperglycemic conditions, conditions involving hyperinsulinemia and conditions involving impaired insulin sensitivity.
56. The peptide or peptidomimetic according to item 55, wherein the condition is selected from the list consisting of type 1 diabetes, type 2 diabetes, type 3 diabetes, metabolic syndrome, prediabetes, obesity, Alzheimer's disease and Cushing's syndrome.

### DETAILED DESCRIPTION

The inventors have studied the role of digestive enzymes, produced by the exocrine pancreas, in glucose metabolism. The inventors have surprisingly found that certain short fragments of amylase are effective, or in some cases significantly more effective, in regulating blood glucose than the amylase from which the one or more fragments are derived from in its intact (non-fragmented) state.

### Effective amylase fragment peptides or peptidomimetics

Peptides were chosen for tests based on premise that the same or very similar peptide for both human and pig possess the same function. Thus, the inventors selected sequences which are identical or highly similar for pig and human. The experiments were to be performed on pigs, so the inventors chose peptides matching the porcine amylase sequence.

It is known that amylase after digestion in stomach loses its activity. Consequently, all peptides were chosen from porcine amylase after its digestion with trypsin+chymotrypsin or just by trypsin.

There is a known peptide called "enterostatin" which has biological activity (influence on fat intake). Enterostatin contains 5 amino-acids, has high pl=10.9 and is rich in hydrophilic amino acids and it is also resistant to digestion by carboxypeptidases A, B.

Thus, the inventors decided to choose relatively short and stable peptides which are resistant to carboxypeptidases A and B. It is known that amylase is relatively resistant to digestion, so they selected peptides which are situated on the flexible parts of polypeptide chain and thus more readily available to digestion (on surface of the molecule).

The inventors hypothesized that the effect by amylase and amylase fragments on glucose metabolism was due to peptide(s) which can be easily released by digestion of amylase molecule by trypsin+chymotrypsin mixture and also tend to be stable enough to reach the surface of enterocytes and elicit the activity.

There were only few basic peptides in simulated trypsin-chymotrypsin digestion and it was hypothesized that one of these peptides serves as specific effector molecule in glucose metabolism (akin to enterostatin). Based on the biochemical properties of peptides (acidic and neutral peptides require pH 7-8 to be dissolved, while the basic peptides require pH around 4-5 for their solubilization (E. Gloaguen , M.Mons. Chapter Gas-Phase IR Spectroscopy and Structure of Biological Molecules. Volume 364 of the series Topics I Current Chemistry pp 225-270 Date: 25 March 2015; http://www.sigmaaldrich.com/technical-documents/articles/biology/peptide-solubility.html) it was hypothesized that neutral and acidic peptides can be easier absorbed by enterocytes (such peptides are more soluble due to basic/neutral pH in the small intestine and thus be available for the enterocytes). It was concluded to design and synthesize one basic peptide with high pl, two acidic peptides, and two neutral ones. The sequences are derived from porcine amylase, but are identical to human sequences except for the residues indicated in **bold** below.

**Table 7: Studied amylase peptides and their sequence differences from human amylase**

| **SEQ ID NO:** | **Sequence** | **pl** | **Comment** |
|---|---|---|---|
| 1 | GNENEFR | pl 4.15 | Neutral, hydrophilic (short fragment just after trypsin digestion) |
| 2 | TSIVHLFEWR | pl 7.5 | Neutral, hydrophobic (short fragment just after trypsin digestion) |
| 3 | SISNSAEDPF | pl 0.7 | Acidic, hydrophilic (trypsin+chymotrypsin, the end of the sequence) |
| 4 | VNGEDVNDW | pl 0.48 | Acidic, hydrophilic (trypsin+chymotrypsin) |
| 5 | IGPPNNNGVIK | pl 10.4 | Basic, hydrophobic (trypsin+chymotrypsin) |

Thus, in a first aspect of the present invention, there is provided an isolated peptide or peptidomimetic of no more than 20 residues, comprising an amylase sequence selected from:
a. GNENEFR (SEQ ID NO:1);
b. SEQ ID NO: 1 with 4N to D substitution;
b. TSIVHLFEWR (SEQ ID NO: 2);
c. SISNSAEDPF (SEQ ID NO: 3);
d. VNGEDVNDW (SEQ ID NO: 4);
e. IGPPNNNGVIK (SEQ ID NO: 5);
f. SEQ ID NO: 4 with 1V to F and/or 4E to N substitution;
f. a sequence having 1, 2, 3 or 4 differences from any of SEQ ID NOs:1-5, wherein the differences are residue substitutions; and
g. a sequence having 1, 2, 3, 4, 5 or 6 differences from any of SEQ ID NOs:1-5, wherein the differences are independently selected from residue substitutions, residue deletions and residue insertions internal to the sequence of any of SEQ ID Nos: 1-5.

The peptide or peptidomimetic is effective, or preferably significantly more effective than intact amylase in regulating glucose homeostasis in a mammal. In particular, the peptide or peptidomimetic reduces the amount of glucose absorbed into bloodstream from the gut, in particular duodenum. Without wishing to be bound by theory, the inventors believe that an increased proportion of the glucose absorbed from the gut by the enterocytes is being utilized by the enterocytes themselves for energy or stored in the enterocytes as glycogen, rather than being transported to bloodstream. Thus, the rest of the body including the liver is exposed to a lower glucose load as a result of administration of the peptide or peptidomimetic. The peptide or peptidomimetic is preferably administered orally or intraduodenally.

By "significantly more" effective in this context is meant that the peptide or peptidomimetic has a greater biological effect in increasing glucose clearance in a mammal after oral administration, compared to intact amylase from which the peptide or peptidomimetic is derived. Preferably, the term "effective" in this context also entails that the peptide or peptidomimetic have a greater biological effect in increasing insulin sensitivity in a mammal after oral administration, compared to intact amylase from which the peptide or peptidomimetic are derived. The comparison is preferably made at an equimolar basis, e.g. one nmol of the peptide or peptidomimetic is compared to one nmol of an intact amylase from which the peptide or peptidomimetic are derived.

*Amylase from which the peptide or peptidomimetic is derived* refers to an amylase comprising the corresponding primary structure(s) as the primary structure of the peptide or peptidomimetic. The peptide or peptidomimetic and the intact amylase may or may not share a common origin or method of manufacture.

By *greater biological effect* is meant that the difference can be detected with generally accepted statistical significance (e.g. p<0.05). The amplitude of the difference may be that the biological effect of the peptide or peptidomimetic is at least 10%, 20%, 30%, 40%, 50%, 60%, 70%, 80%, 90%, 100%, 120%, 150%, 200%, or 300% higher than that of the intact amylase.

The peptide or peptidomimetic is preferably regarded as effective when they have a statistically significantly greater effect on glucose clearance in an intraduodenal glucose tolerance test compared to intact amylase from which the peptide or peptidomimetic is derived.

The amylase sequence may be a sequence having 1, 2 or 3 differences from any of SEQ ID NOs:1-5, wherein the differences are independently selected from residue substitutions, residue deletions and residue insertions internal to the sequence of any of SEQ ID NOs: 1-5.

The amylase sequence may be a sequence having 1, 2 or 3 differences from any of SEQ ID NOs:1-5, wherein the differences are independently selected from residue deletions and residue insertions internal to the sequence of any of SEQ ID Nos: 1-5.

The amylase sequence may have a sequence differing from any of SEQ ID NO: 1-5 by alanine substitutions numbering 1, 2, 3, 4, 5 or 6 in total.

In certain preferred embodiments, the amylase sequence of the first aspect may be selected from:
a. GNENEFR (SEQ ID NO: 1);
b. SEQ ID NO: 1 with 4N to D substitution;
c. a sequence having 1, 2, 3 or 4 differences from SEQ ID NO:1, wherein the differences are residue substitutions; and
d. a sequence having 1, 2, 3, 4, 5 or 6 differences from SEQ ID NO:1, wherein the differences are independently selected from residue substitutions, residue deletions and residue insertions internal to the sequence of SEQ ID NO:1.

In certain preferred embodiments, the amylase sequence of the first aspect may be selected from:
a. TSIVHLFEWR (SEQ ID NO: 2);
b. a sequence having 1, 2, 3 or 4 differences from SEQ ID NO: 2, wherein the differences are residue substitutions; and
c. a sequence having 1, 2, 3, 4, 5 or 6 differences from SEQ ID NO: 2, wherein the differences are independently selected from residue substitutions, residue deletions and residue insertions internal to the sequence of SEQ ID NO: 2.

In certain preferred embodiments, the amylase sequence of the first aspect may be selected from:
a. SISNSAEDPF (SEQ ID NO: 3);
b. a sequence having 1, 2, 3 or 4 differences from SEQ ID NO: 3, wherein the differences are residue substitutions; and
c. a sequence having 1, 2, 3, 4, 5 or 6 differences from SEQ ID NO: 3, wherein the differences are independently selected from residue substitutions, residue deletions and residue insertions internal to the sequence of SEQ ID NO: 3.

In certain preferred embodiments, the amylase sequence of the first aspect may be selected from:
a. VNGEDVNDW (SEQ ID NO: 4);
b. SEQ ID NO: 4 with 1V to F and/or 4E to N substitution;
c. a sequence having 1, 2, 3 or 4 differences from SEQ ID NO: 4, wherein the differences are residue substitutions; and
d. a sequence having 1, 2, 3, 4, 5 or 6 differences from SEQ ID NO: 4, wherein the differences are independently selected from residue substitutions, residue deletions and residue insertions internal to the sequence of SEQ ID NO: 4.

In certain preferred embodiments, the amylase sequence of the first aspect may be selected from:
a. IGPPNNNGVIK (SEQ ID NO: 5);
b. a sequence having 1, 2, 3 or 4 differences from SEQ ID NO:5, wherein the differences are residue substitutions; and
c. a sequence having 1, 2, 3, 4, 5 or 6 differences from SEQ ID NO:5, wherein the differences are independently selected from residue substitutions, residue deletions and residue insertions internal to the sequence of SEQ ID NO:5.

In certain preferred embodiments, the amylase sequence of the first aspect may be GNENEFR (SEQ ID NO:1) or SEQ ID NO: 1 with 4N to D substitution. In certain preferred embodiments, the amylase sequence of the first aspect may be TSIVHLFEWR (SEQ ID NO: 2). In certain preferred embodiments, the amylase sequence of the first aspect may be SISNSAEDPF (SEQ ID NO: 3). In certain preferred embodiments, the amylase sequence of the first aspect may be VNGEDVNDW (SEQ ID NO: 4) or SEQ ID NO: 4 with 1V to F and/or 4E to N substitution. In certain preferred embodiments, the amylase sequence of the first aspect may be IGPPNNNGVIK (SEQ ID NO: 5).

In certain preferred embodiments, the peptide or peptidomimetic of the first aspect may have the sequence GNENEFR (SEQ ID NO:1) or SEQ ID NO: 1 with 4N to D substitution. In certain preferred embodiments, the peptide or peptidomimetic of the first aspect may have the sequence TSIVHLFEWR (SEQ ID NO: 2). In certain preferred embodiments, the peptide or peptidomimetic of the first aspect may have the sequence SISNSAEDPF (SEQ ID NO: 3). In certain preferred embodiments, the peptide or peptidomimetic of the first aspect may have the sequence VNGEDVNDW (SEQ ID NO: 4) or SEQ ID NO: 4 with 1V to F and/or 4E to N substitution. In certain preferred embodiments, the peptide or peptidomimetic of the first aspect may have the sequence IGPPNNNGVIK (SEQ ID NO: 5).

Preferably, the peptide or peptidomimetic of the first aspect may have 11, 12, 13, 14, 15, 16, 17, 18, 19 or 20 residues.

Preferably, the peptide or peptidomimetic of the first aspect may have no more than 18, more preferably no more than 15 residues, most preferably no more than 12.

Preferably, the peptide or peptidomimetic of the first aspect is a peptide.

### Modifications

Preferably, the peptide or peptidomimetic of the first aspect is a peptidomimetic.

The peptide or peptidomimetic according to the first aspect may have a modified C-terminal, preferably an amidated C-terminal.

The peptide or peptidomimetic according to the first aspect may have a modified N-terminal, preferably an acylated N-terminal.

The peptide or peptidomimetic according to the first aspect may have a cyclic backbone.

The peptide or peptidomimetic according to the first aspect may comprise one or more non-natural residues.

The peptide or peptidomimetic according to the first aspect may comprise one or more D-amino acid residues.

The peptide or peptidomimetic according to the first aspect may comprise one or more non-natural bonds in place of peptide bonds.

### Non-natural peptides

Preferably, the peptide or peptidomimetic according to the first aspect is a peptide and comprises at least one difference compared to any naturally occurring peptide.

Preferably, the peptide or peptidomimetic according to the first aspect is a peptide wherein the sequence of the peptide comprises at least one difference compared to any of the sequences according to SEQ ID NO: 1-5.

### Composition

In a second aspect, there is provided a composition comprising a peptide or peptidomimetic of the first aspect, or more than one such peptides or peptidomimetics. The composition may comprise 1, 2, 3, 4 or 5 peptides or peptidomimetics each having a sequence comprising an amylase sequence based on any of SEQ ID NOs:1-5, having sequences and features according to the first aspect. Preferably, the composition comprises 2, 3, 4 or 5 peptides or peptidomimetics each having a sequence comprising an amylase sequence based on a different sequence selected from any of SEQ ID NOs:1-5 as discussed above. Preferably, the composition comprises 5 peptides according to SEQ ID NOs: 1-5.

The composition may have any formulation, and can also be included in foodstuffs. The composition may be a pharmaceutical composition further comprising a pharmaceutically acceptable carrier. The composition may be a dietary supplement or a nutraceutical. The composition may further comprise a calorie source and may be a medical food, a functional food or a part thereof.

The composition may be formulated as a powder, a tablet, a capsule, a liquid, as a part of a liquid food item, or as a part of a solid food item.

The composition may be as an enterically coated formulation. Preferably, the composition is formulated for release in the duodenum, to avoid exposing the amylase fragments to conditions in the stomach that might degrade efficacy. Suitable delayed-release formulations of the kind are well known in the art for instance from US 8562981 B2 or EP 0261213 B1.

### Conditions for treatment and prevention

Since the peptide or peptidomimetic of the first aspect (see Example 1) reduces glucose load in portal and jugular vein following intraduodenal glucose administration, the peptide or peptidomimetic of the first aspect is useful as a medicament for treatment or prevention of a number of pathological conditions.

Thus, the peptide or peptidomimetic of the first aspect or the composition of the second aspect may be for use in the treatment or prevention of a condition selected from the list consisting of hyperglycemic conditions, conditions involving hyperinsulinemia and conditions involving impaired insulin sensitivity.

The above-mentioned condition may be a condition selected from the list consisting of type 1 diabetes, type 2 diabetes, type 3 diabetes, metabolic syndrome, prediabetes, obesity, Alzheimer's disease and Cushing's syndrome.

An appropriate dosing regimen is within the skill of the treating physician and depends on the nature of the disease and the individual characteristics of the patient to be treated. Based on the present results on pigs, an appropriate dosing regime may be 0.05 g/kg body weight /3 times daily, preferably 1 h -30 min before meal (or with meal).

### General aspects of the present disclosure

The term "comprising" is to be interpreted as including, but not being limited to. All references are hereby incorporated by reference. The arrangement of the present disclosure into sections with headings and subheadings is merely to improve legibility and is not to be interpreted limiting in any way, in particular, the division does not in any way preclude or limit combining features under different headings and subheadings with each other.

### EXAMPLES

The following examples are not to be regarded as limiting. For further information on the experimental details, the skilled reader is directed to a separate section titled Materials and Methods.

### Example 1: Effect of a set of specific amylase peptides in intraduodenal glucose tolerance test

Intraduodenal glucose tolerance test (ID.GTT) was chosen as convenient and appropriate model for testing glucose absorption and glucose-stimulated insulin response together with the capacity for blood glucose clearance. Young pigs were used as the experimental model because of their physiological similarity to humans, good reproducibility, and ability to be trained, which decreases stress under experimental conditions

After pure IDGTT in bariatric pigs the glucose level in peripheral and portal blood rose and were higher than in control pigs, suggesting that bariatric pigs duodenum is permeable for more glucose than duodenum of control pigs. An additional consideration is that portal blood can account for 40% of total heart rate flow, which could be taken to suggest reduced liver metabolism of glucose in bariatric pigs.

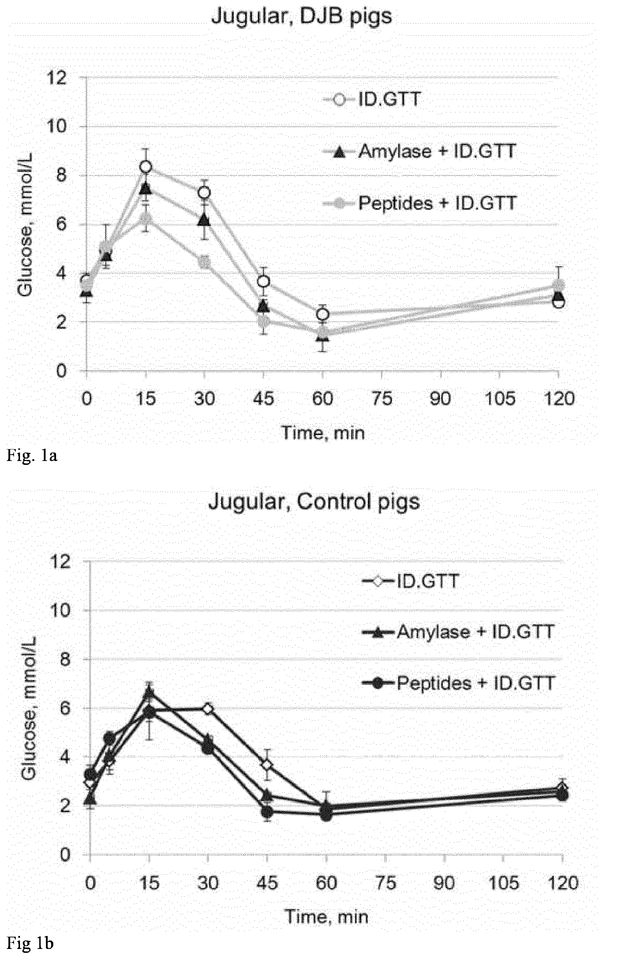

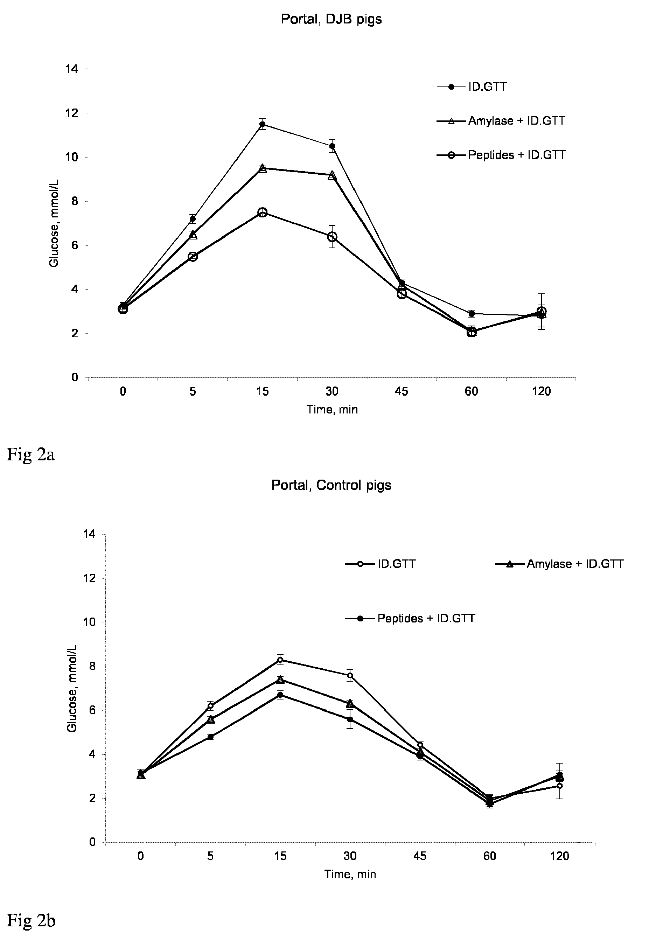

In general, glucose utilization during IDGTT had a similar time pattern both in bariatric (DJB) and control pigs and after 120 minutes, blood glucose level (jugular and portal veins) was reverted to the initial values (Figures 1, 2, Tables 1, 2). This shows that all loaded glucose is eliminated from intestine and blood in a very similar time frame both in normal and DJB pigs.

Intraduodenal administration of both amylase and mixture of chosen peptides together with glucose during IDGTT decreased blood glucose clearance from gut and/or appearance in blood both in jugular and portal veins both in DJB and Control groups. However, effect of peptides was significantly higher than that of intact amylase, on a roughly equimolar basis (the intact amylase preparation only contains about 10 wt.% amylase enzyme). Peptides provided the strongest decrease in blood glucose levels in both experimental groups.

Intraduodenal administration of both amylase and mixture of chosen peptides affected blood glucose clearance, both amylase and isolated peptides caused lower blood glucose level already at 5 minutes after glucose loading, compared to ID.GTT without any additive (p<0.001). However, peptides exhibited significantly more potent lowering effect since post loading values at 5, 15 and 30 min for glucose were significantly lower for isolated peptides as compared to amylase in both DJB and Control pigs (p<0.001). (Data for jugular and portal veins, Figures 1 and 2)

AUC glucose is informative of the sum of glucose utilization during IDGTT in pigs after the administration of different treatments (Tables 1 and 2, Figures 1 and 2). There was a significant influence on the total blood glucose utilization of both amylase and peptides, but the effect of peptides was significantly higher than that of amylase (jugular and portal veins).

**TABLE 1: Portal glucose concentration, control pigs**

| Glucose concentration in portal blood, mmol/L control pigs | | | | | | | | AUC area under the curve |
|---|---|---|---|---|---|---|---|---|
| Time, min | | | | | | | | |
| | 0 | 5 | 15 | 30 | 45 | 60 | 120 | |
| DGTT Pep | 3.15±0.17a | 4.80±0.12b | 6.70±0.19e | 5.60±0.43c | 3.90±0.16h | 1.75±0.18k | 3.07±0.53a | 427.9±9.04a |
| DGTT Am | 3.06±0.13a | 5.60±0.14c | 7.40±0.14f | 6.30±0.15d | 4.10±0.18h | 1.90±0.27k | 3.00±0.24a | 459.4±11.35b |
| DGTT | 3.10±0.09a | 6.20±0.21d | 8.30±0.23g | 7.59±0.27f | 4.43±0.14i | 2.01±0.13k | 2.56±0.57a | 490.5±19.07c |

**TABLE 2: Portal glucose concentration, DJB pigs**

| Glucose concentration in portal blood, mmol/L - bariatric pigs | | | | | | | | AUC area under the curve |
|---|---|---|---|---|---|---|---|---|
| Time, min | | | | | | | | |
| | 0 | 5 | 15 | 30 | 45 | 60 | 120 | |
| DGTT Pep | 3.12±0.21a | 5.50±0.10b | 7.50±0.20d | 6.40±0.50c | 3.80±0.16g | 2.10±0.21i | 3.00±0.80a | 620.80±25.31a |
| DGTT Am | 3.21±0.11a | 6.50±0.15c | 9.51±0.10e | 9.20±0.10e | 4.20±0.16h | 2.12±0.24i | 2.95±0.21a | 544.70+34.57b |
| DGTT | 3.30±0.10a | 7.20±0.20d | 11.50±0.25f | 10.50±0.30f | 4.30±0.17h | 2.90±0.15i | 2.8±0.50a | 464.60±23.45c |

In the tables, mean values with a letter (such as a, b or c) are statistically significantly different from values in other groups **not** denoted with the same letter (P <0,05).

Taken together, the above results suggest that:
1. The bariatric model used is a highly sensitive tool to describe participation of intestine vs. peripheral metabolism to utilise diet glucose.
2. The bariatric model used allows for the first time one to observe that amylase can stimulate glucose utilisation and/or storage by gut mucosa or gut per se.
3. The peptides being components of amylase of most of vertebrates down regulate glucose absorption from intestine and tremendously increase glucose utilisation by gut per se or gut mucosa.
4. Liver metabolism cannot be main responsible for the observed glucose elimination during IDGTT.

Conclusions:
1. The peptides used are potent factors to lower absorption of glucose into bloodstream thus, they are anti-diabetic type II and anti-obesity factors since absorbed glucose is mostly converted to fat in obese patients.
2. Amylases -both pancreatic and salivary can be factors down regulating obesity and development of diabetes type II since used peptides are always components of amino-acids chain in the both amylases

### MATERIALS AND METHODS

### Animals

The study was approved by the Malmö/Lund Ethical Committee on Animal Experiments (Sweden). The experiments were performed on crossbred ((Yorkshire x Swedish Landrace) x Hampshire) pigs. All pigs were housed individually in pens in the same stable at 20 ± 2°C and with light on from 7.00-19.00. The pigs had free access to water via a drinking nipple and were offered a commercial pelleted feed (Växtill 320, Lantmännen, Sweden), 4% of their body weight per day (at 11.00-12.00). Pigs were socialized to prevent stress during manipulation and the experiments. At the age of 8 weeks, 6 weaned pigs (10 ± 2 kg) were selected for experiment.

### Surgery

Bariatric surgery resulting in construction of: biliopancreatic limb (100 - 120 cm), alimentary limp 100 -120 cm and common limb - the rest o the intestine after junction of biliopancratic and alimentary limb, In brief. After an overnight fasting period the pigs were pre-medicated with azaperone (Stresnil^{®}, Janssen Pharmaceutica, Belgium, 2.2 mg/kg, intramuscularly) and washed using surgical soap. The pigs were then anaesthetized via an inhalation mask with a 0.5-1.5% air mixture of Fluothane^{®} (Astra Läkemedel, Södertälje, Sweden) in O₂ as a carrier gas at approximately 0.5-1 L/min, using a close-circuit respiratory flow system (Komesaroff Medical Developments, Melbourne, Australia). Post-operative pain was prevented by administration of buprenorphine (Temgesic^{®}, Schering-Plough AB, 0.01 mg/kg, intramuscularly) for 1 day. Ampicillin (Doktacillin, Astra Läkemedel, Södertälje, Sweden) was administrated i.v. (15 mg/kg) and at the incision site (250-500 mg). Sham-operated animals were used as controls.

Catheters for intestinal infusions were placed into biliopancreatic. Alimentary and common limb limbs in their beginnings. Sham-operated pigs got catheters into respective parts of the intestine - duodenum and jejunum.

Jugular and portal catheters were in operated for blood collection during the experimental period to control exocrine (pancreatic enzymes presence) and endocrine (insulin, C-peptide levels) functions and nutritional status (glucose level).

### Experiments and design

In control experiment only IDGTT (intraduodenal glucose tolerance test) (50% glucose, 1g/kg b wt, infused during 30 sec) test was performed both in DJB and Control pigs.

The peptides were synthesized by GL Biochem Ltd. (Shanghai, China).

*Peptides* (mix of 5 mentioned peptides, 50 mg/kg body weight) were dissolved in 50% glucose solution and uploaded via catheter to biliopancreatic/ limb or into duodenum during IDGTT.

Amylase porcine enzyme (Sigma-Aldrich *A3176 α-Amylase from porcine pancreas Type VI-B, ≥10 units*/*mg solid)* was used.

The dose of the investigated enzyme was correlated to the 4 pancrelipase capsules (Creon^{®} 10,000, Abbott Healthcare Products Ltd, Southampton, United Kingdom) and based on the corresponding amylolytic activity were used as a single dose /pig (approx. 20 kg).

The intact amylase was administered at 500 mg/kg (calculated as 10 % of amylase powder administered).

The combined amount of the 5 peptides administered was 50 mg/kg.

Given the fact that porcine amylase consists of 511 amino acids and the peptides contain in total 47 amino acids, the amounts required for equimolar proportion would be approximately 47 : 517. Therefore, the respective administered amounts 50 mg/kg and 500 mg/kg are approximately equimolar. Please note that one amylase molecule one can as a theoretical maximum produce only one molecule of any of the selected peptides or one set of selected peptides through digestion by proteases.

Amylase, as well as peptides were loaded together with 50% glucose solution (1g/kg bwt) during 30 seconds via catheters to biliopancreatic limb in DJB pigs or to the duodenum in Control pigs. Immediately after infusion, the catheter was flushed with 5ml of 0,9% sterile saline solution.

Blood samples for the glucose tolerance test were withdrawn at -1 (presented as time point 0 before infusion), 5, 15, 30, 45, 60, 120 minutes after infusion. Blood was collected into 5 ml syringes containing EDTA (0.20 mg) and a protease inhibitor, aprotinin (1000 kIU, Bayer, Leverkusen, Germany), as described

### Analysis of blood glucose

Glucose was measured in the fresh blood samples using a glucose-meter with test strips (Accu-Chek^{®} Aviva, Roche Diagnostics, Mannheim, Germany).

### Statistical analysis and calculations

Statistical data analyses were done using Prism, version 6 (GraphPad Software, Inc, San Diego, CA, USA). The level of statistical significance between time points was determined using repeated measures two-way Anova with Tukey's multiple comparison test. The differences for the AUC were determined using two-way Anova with Sidak's multiple comparison tests. A P-value of < 0.05 was considered to be statistically significant while a P < 0.1 as a tendency.

### REFERENCES

Bays, Harold E 2004 Current and investigational antiobesity agents and obesity therapeutic treatment targets. Obesity research 12(8):1197-1211.
Christoffersen, Berit, et al. 2009 Evaluation of different methods for assessment of insulin sensitivity in Göttingen minipigs: introduction of a new, simpler method. American Journal of Physiology-Regulatory, Integrative and Comparative Physiology 297(4):R1195-R1201.
Ewald, Nils and Philip Hardt. Alterations in Exocrine Pancreatic Function in Diabetes Mellitus. Pancreapedia. February 2015 DOI: 10.3998/panc.2015.7.
Ewald, Nils, and Philip D Hardt 2013 Diagnosis and treatment of diabetes mellitus in chronic pancreatitis. World journal of gastroenterology: WJG 19(42):7276.
Ewald, Nils, et al. 2007 Pancreatin therapy in patients with insulin-treated diabetes mellitus and exocrine pancreatic insufficiency according to low fecal elastase 1 concentrations. Results of a prospective multi-centre trial. Diabetes/metabolism research and reviews 23(5):386-391.
Falchi Mario et al. Low copy number of the salivary amylase gene predisposes to obesity. Nature Genetics 2014. 46 (5); pp 492-497.
Fedkiv, Olexandr, et al. 2009 Growth is dependent on the exocrine pancreas function in young weaners but not in growing-finishing pigs. Journal of physiology and pharmacology 60(3):55-59.
Gavini et al. Composition and method for modulating inflammatory molecules with amylase United States Patent Application Publication Pub. No.: US 2013/0273026 A1 Oct. 17, 2013 .
Glasbrenner, B, et al. 1990 Effect of pancreatin on diabetes mellitus in chronic pancreatitis]. Zeitschrift fur Gastroenterologie 28(6):275.
Gloaguen E and Mons M. Isolated Neutral Peptides. - Chapter Gas-Phase IR Spectroscopy and Structure of Biological Molecules. Volume 364 of the series Topics I Current Chemistry pp 225-270 Date: 25 March 2015;
Granger, M, Abadie, B. and G. Marchis-Mouren. Limited action of trypsin on porcine pancreatic amylase : characterization of the fragments. FEBS Letters, 1975. 56(2); pp 189-193.
Hardt, Philip and N. Ewald. Exocrine Pancreatic Insufficiency in Diabetes Mellitus: A Complication of Diabetic Neuropathy or a Different Type of Diabetes? Experimental Diabetes Research Volume 2011, Article ID 761950, 7 pages. http://www.sigmaaldrich.com/technical-documents/articles/biology/peptide-solubility.html)
Knop, Filip K, et al. 2007 Increased postprandial responses of GLP-1 and GIP in patients with chronic pancreatitis and steatorrhea following pancreatic enzyme substitution. American Journal of Physiology-Endocrinology and Metabolism 292(1):E324-E330.
Lozinska, Liudmyla, et al. 2013 Impact of pancreatic enzymes on blood glucose and insulin release in a porcine model. Pancreatology 13(3):S64.
McDougall, Gordon J, and Derek Stewart 2005 The inhibitory effects of berry polyphenols on digestive enzymes. Biofactors 23(4):189-195.
Mohan, V, S Poongothai, and CS Pitchumoni 1998 Oral pancreatic enzyme therapy in the control of diabetes mellitus in tropical calculous pancreatitis. International journal of pancreatology 24(1):19-22.
Nakajima Kei. Low serum amylase and obesity, diabetes and metabolic syndrome: A novel interpretation. World J Diabetes. 2016 Mar 25;7(6):112-21.
O'Keefe, Stephen JD, Abdool K Cariem, and Margaret Levy 2001 The exacerbation of pancreatic endocrine dysfunction by potent pancreatic exocrine supplements in patients with chronic pancreatitis. Journal of clinical gastroenterology 32(4):319-323.
Rantzer, D, J Svendsen, and B Weström 1995 Weaning of pigs raised in sow-controlled and in conventional housing systems, 1: Description of systems, production and bacteriology. Swedish Journal of Agricultural Research 25.
Rengman, Sofia, et al. 2009 An elemental diet fed, enteral or parenteral, does not support growth in young pigs with exocrine pancreatic insufficiency. Clinical nutrition 28(3):325-330.
Salt, W and Schenker S. Amylase - its clinical significance: a review of the literature. Medicine. 1976, 55 (4), pp 269-289.

## Claims

1. An isolated peptide or peptidomimetic of no more than 20 residues,
comprising an amylase sequence selected from:
a. GNENEFR (SEQ ID NO:1);
b. SEQ ID NO: 1 with 4N to D substitution;
c. TSIVHLFEWR (SEQ ID NO: 2);
d. SISNSAEDPF (SEQ ID NO: 3);
e. VNGEDVNDW (SEQ ID NO: 4);
f. SEQ ID NO: 4 with 1V to F and/or 4E to N substitution;
g. IGPPNNNGVIK (SEQ ID NO: 5);
h. a sequence having 1, 2, 3 or 4 differences from any of SEQ ID NOs:1-5, wherein the differences are residue substitutions; and
i. a sequence having 1, 2, 3, 4, 5 or 6 differences from any of SEQ ID NOs:1-5, wherein the differences are independently selected from residue substitutions, residue deletions and residue insertions internal to the sequence of any of SEQ ID NOs: 1-5.

2. The peptide or peptidomimetic according to claim 1, wherein the amylase sequence is a sequence having 1, 2 or 3 differences from any of SEQ ID NOs:1-5, wherein the differences are independently selected from residue substitutions, residue deletions and residue insertions internal to the sequence of any of SEQ ID Nos: 1-5.

3. The peptide or peptidomimetic according to any of claims 1-2, wherein the amylase sequence is a sequence having 1, 2 or 3 differences from any of SEQ ID NOs:1-5, wherein the differences are independently selected from residue deletions and residue insertions internal to the sequence of any of SEQ ID Nos: 1-5.

4. The peptide or peptidomimetic according to any of claims 1-3, wherein said amylase sequence has a sequence differing from any of SEQ ID NO: 1-5 by alanine substitutions numbering 1, 2, 3, 4, 5 or 6 in total.

5. The peptide or peptidomimetic according to any of claims 1-4, comprising no more than 15 residues.

6. The peptide or peptidomimetic according to any of claims 1-5, comprising no more than 12 residues.

7. The peptide or peptidomimetic according to any of claims 1-6, wherein the peptide or peptidomimetic is effective in regulating glucose homeostasis in a mammal.

8. The peptide or peptidomimetic according to any of claims 1-7, for use as a medicament.

9. The peptide or peptidomimetic according to any of claims 1-8, for use in the treatment or prevention of condition selected from the list consisting of hyperglycemic conditions, conditions involving hyperinsulinemia and conditions involving impaired insulin sensitivity.

10. The peptide or peptidomimetic according to claim 9, wherein the condition is selected from the list consisting of type 1 diabetes, type 2 diabetes, type 3 diabetes, metabolic syndrome, prediabetes, obesity, Alzheimer's disease and Cushing's syndrome.

11. A composition for oral use, comprising a peptide or peptidomimetic according to any of claims 1-10.

12. A composition according to claim 11, comprising the following five peptides or peptidomimetics of no more than 20 residues:
a. a peptide or peptidomimetic comprising an amylase sequence selected from:
i. GNENEFR (SEQ ID NO: 1);
ii. SEQ ID NO: 1 with 4N to D substitution;
iii. a sequence having 1, 2, 3 or 4 differences from SEQ ID NO:1, wherein the differences are residue substitutions; and
iv. a sequence having 1, 2, 3, 4, 5 or 6 differences from SEQ ID NO:1, wherein the differences are independently selected from residue substitutions, residue deletions and residue insertions internal to the sequence of SEQ ID NO:1.
b. a peptide or peptidomimetic comprising an amylase sequence selected from:
i. TSIVHLFEWR (SEQ ID NO: 2);
ii. a sequence having 1, 2, 3 or 4 differences from SEQ ID NO: 2, wherein the differences are residue substitutions; and
iii. a sequence having 1, 2, 3, 4, 5 or 6 differences from SEQ ID NO: 2, wherein the differences are independently selected from residue substitutions, residue deletions and residue insertions internal to the sequence of SEQ ID NO: 2.
c. a peptide or peptidomimetic comprising an amylase sequence selected from:
i. SISNSAEDPF (SEQ ID NO: 3);
ii. a sequence having 1, 2, 3 or 4 differences from SEQ ID NO: 3, wherein the differences are residue substitutions; and
iii. a sequence having 1, 2, 3, 4, 5 or 6 differences from SEQ ID NO: 3, wherein the differences are independently selected from residue substitutions, residue deletions and residue insertions internal to the sequence of SEQ ID NO: 3;
d. a peptide or peptidomimetic comprising an amylase sequence selected from:
i. VNGEDVNDW (SEQ ID NO: 4);
ii. SEQ ID NO: 4 with 1V to F and/or 4E to N substitution;
iii. a sequence having 1, 2, 3 or 4 differences from SEQ ID NO: 4, wherein the differences are residue substitutions; and
iv. a sequence having 1, 2, 3, 4, 5 or 6 differences from SEQ ID NO: 4, wherein the differences are independently selected from residue substitutions, residue deletions and residue insertions internal to the sequence of SEQ ID NO: 4; and
e. a peptide or peptidomimetic comprising an amylase sequence selected from:
i. IGPPNNNGVIK (SEQ ID NO: 5);
ii. a sequence having 1, 2, 3 or 4 differences from SEQ ID NO:5, wherein the differences are residue substitutions; and
iii. a sequence having 1, 2, 3, 4, 5 or 6 differences from SEQ ID NO:5, wherein the differences are independently selected from residue substitutions, residue deletions and residue insertions internal to the sequence of SEQ ID NO:5.

13. The composition according to claim 12, wherein the composition comprises the following peptides:
a. a peptide, the sequence of which consists of GNENEFR (SEQ ID NO:1) or SEQ ID NO: 1 with substitution of 4N to D;
b. a peptide, the sequence of which consists of TSIVHLFEWR (SEQ ID NO: 2);
c. a peptide, the sequence of which consists of SISNSAEDPF (SEQ ID NO: 3);
d. a peptide, the sequence of which consists of VNGEDVNDW (SEQ ID NO: 4) or SEQ ID NO: 4 with 1V to F and/or 4E to N substitution; and
e. a peptide, the sequence of which consists of IGPPNNNGVIK (SEQ ID NO: 5).

14. The composition according to any of claims 11-13, wherein the composition is formulated for duodenal release.

15. The composition according to any of claims 11-14, for use as a medicament.


SEQUENCE LISTING

<110>  CENTRUM INNOWACJI EDORADCA SP. Z O. O. SPÓLKA KOMANDYTOWA

<120>  BIOACTIVE PEPTIDES FOR BLOOD GLUCOSE CONTROL

<130>  NP0254EP-P

<160>  5

<170>  PatentIn version 3.5

<210>  1
<211>  7
<212>  PRT
<213>  Sus scrofa

<400>  1

Gly Asn Glu Asn Glu Phe Arg
1               5

<210>  2
<211>  10
<212>  PRT
<213>  Sus scrofa

<400>  2

Thr Ser Ile Val His Leu Phe Glu Trp Arg
1               5                   10

<210>  3
<211>  10
<212>  PRT
<213>  Sus scrofa

<400>  3

Ser Ile Ser Asn Ser Ala Glu Asp Pro Phe
1               5                   10

<210>  4
<211>  9
<212>  PRT
<213>  Sus scrofa

<400>  4

Val Asn Gly Glu Asp Val Asn Asp Trp
1               5

<210>  5
<211>  11
<212>  PRT
<213>  Sus scrofa

<400>  5

Ile Gly Pro Pro Asn Asn Asn Gly Val Ile Lys
1               5                   10